# EUROPÄISCHE PATENTSCHRIFT

(11) **EP 2 563 280 B1**
(45) Veröffentlichungstag und Bekanntmachung des Hinweises auf die Patenterteilung: **22.06.2016**
(21) Anmeldenummer: 11717609.9
(22) Anmeldetag: 29.04.2011
(51) Int. Cl.: A61F 2/34, A61F 2/30

(54) **ERHÖHUNG DER BRUCHLAST KERAMISCHER PFANNENEINSÄTZE FÜR HÜFTGELENKPROTHESEN DURCH DEFINIERTE RÜCKSEITENKOLLISION VON PFANNENEINSATZ UND HÜFTPFANNE**
INCREASING THE BREAKING LOAD OF CERAMIC CUP INSERTS FOR HIP JOINT PROSTHESES BY A DEFINED BACK SIDE COLLISION OF THE CUP INSERT AND ACETABULAR CUP
AUGMENTATION DE LA CHARGE DE RUPTURE D'INSERTS DE CAVITÉ COTYLOÏDE EN CÉRAMIQUE POUR DES PROTHÈSES D'ARTICULATION DE LA HANCHE PAR CONTACT ARRIÈRE DÉFINI ENTRE L'INSERT DE CAVITÉ COTYLOÏDE ET LA CAVITÉ COTYLOÏDE

(30) Priorität: 16.07.2010 DE 102010031438; 30.04.2010 DE 102010028402
(43) Veröffentlichungstag der Anmeldung: 06.03.2013
(73) Patentinhaber: CeramTec GmbH, 73207 Plochingen (DE)
(72) Erfinder: PREUSS, Roman, 73230 Kirchheim unter Teck (DE); PANDORF,Thomas, 73249 Wernau (DE)
(74) Vertreter: Scherzberg, Andreas Hans
(86) Internationale Anmeldenummer: PCT/EP2011/056838
(87) Internationale Veröffentlichungsnummer: WO 2011/135074

(56) Entgegenhaltungen:
- EP-A1- 1 025 814
- EP-A1- 1 066 806
- EP-A2- 0 649 641
- EP-A2- 0 796 598
- EP-A2- 1 442 727
- WO-A2-2008/015286
- DE-A1- 19 813 074
- DE-C1- 19 654 409
- US-A- 5 919 236

## Beschreibung

Die Erfindung betrifft eine Hüftpfanne und Pfanneneinsatz für eine Hüftgelenkprothese, wobei der Pfanneneinsatz mit der Hüftpfanne über einen Klemmkonus einer konischen Klemmverbindung im äquatorialen Bereich der beiden Komponenten gekoppelt ist und unterhalb des Klemmkonus bis hin zum Pol ein Spalt zwischen den beiden Komponenten angeordnet ist, der von den radialen Konturzügen beider Komponenten begrenzt ist.

Für moderne Hüftgelenkprothesen werden verschiedene Materialien zur Realisierung einer biokompatiblen, verschleißarmen Lagerung verwendet. Dabei sind die sogenannten Hart-Hart-Paarungen gemäß Stand der Technik für eine dauerhaft-zuverlässige Versorgung des Patienten am besten geeignet. Bei diesen Paarungen bestehen sowohl der auf dem Hüftschaft montierte Kugelkopf als auch der in der Hüftpfanne montierte Pfanneneinsatz aus einem im technischen Sinn harten Material. Es finden derzeit die Paarungen Keramik-Keramik und Metall-Metall Anwendung. Aktuelle Studien untersuchen auch die klinischen Auswirkungen der Paarung Keramik-Metall.

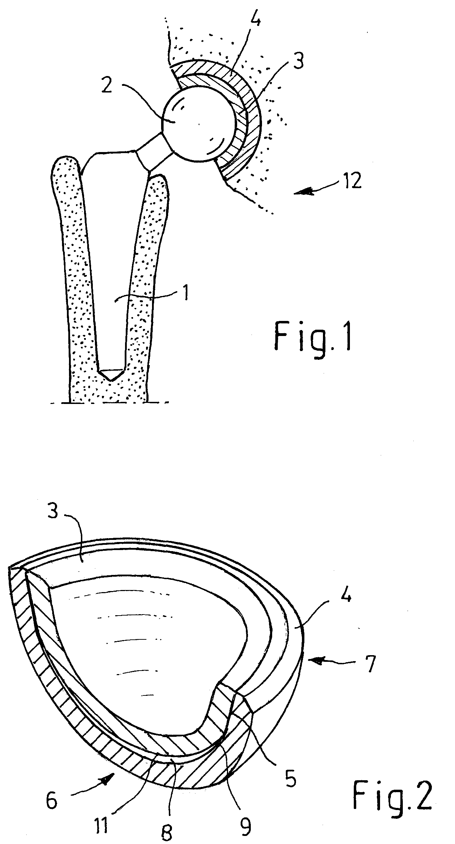

Um dem anwendenden Operateur die Möglichkeit zu geben, intra-operativ die optimale Gleitpaarung für den Patienten auszuwählen, sind moderne Hüftgelenkprothesen modular aufgebaut (siehe Figur 1). Die Implantate bestehen in der Regel aus einem Schaft 1 gekoppelt mit einem Kugelkopf 2 und einer Hüftpfanne 4 gekoppelt mit einem Pfanneneinsatz 3. Schaft und Hüftpfanne sind in der Regel aus Metalllegierungen und mit dem Körper durch Einwachsen in den Oberschenkel- bzw. Beckenknochen verbunden. Sie sind Träger für den Kugelkopf bzw. Pfanneneinsatz. Der Kugelkopf ist in der Kalotte des Pfanneneinsatzes drehbar gelagert mit dem Freiheitsgrad eins.

Die Kopplung zwischen Pfanneneinsatz und Hüftpfanne erfolgt bei direkter Kopplung ohne den Einsatz eines Adaptermaterials, z.B. Kunststoff, und in der Regel durch eine konische Klemmverbindung. Lösungen dafür sind in z.B. in EP 0 649 641, EP 0 826 347 und DE 196 54 409 beschrieben. Dabei fällt auf, dass die Kopplung der Komponenten zumeist durch einen im so genannten äquatorialen Bereich der Hüftpfanne liegenden Klemmkonus erfolgt. Zur Erläuterung des Begriffs "äquatorial" siehe Figur 2, Bezugszeichen 5. Im sogenannten polaren Bereich der Komponenten erfolgt kein Kontakt, stattdessen wird ein signifikanter Spalt vorgesehen, um einen Kontakt der Komponenten in diesem Bereich zu vermeiden - siehe EP 0 649 641 Figur 1 bzw. EP 0 826 347, Figuren 1 bis 4. Zur Erläuterung des Begriffs "polar" siehe Figur 2, Bezugszeichen 6.

Dokument EP 1 025 814 zeigt die Merkmale des Oberbegriffs des unabhängigen Anspruchs 1, siehe insbesondere Figur 2.

Die Notwendigkeit der Vermeidung von Kontakt der Komponenten im rückseitigen Bereich des Pfanneneinsatzes - Geometriebereich unterhalb des Klemmkonus bis hin zum Pol - ergibt sich wie folgt:
1. Durch Belastung des Pfanneneinsatzes erfolgt ein lastabhängiges geringfügiges Einsinken des Pfanneneinsatzes in die Hüftpfanne. Tritt dabei frühzeitig Kontakt zwischen den Komponenten im rückseitigen Bereich auf, führt dies zur Entlastung des Klemmkonus. Dadurch steigt die Klemmkraft der Kopplung nicht weiter an, sondern verharrt auf möglicherweise zu niedrigem Niveau. Werden dann durch Adhäsion zwischen Kugelkopf und Pfanneneinsatz Zugkräfte auf den Pfanneneinsatz übertragen, kann dieser aus der Hüftpfanne gehoben werden.
   Diese Störung der Kopplung kann schließlich in-vivo zum Versagen des Prothesensystems führen. Besteht hingegen ein ausreichender Spalt zwischen den Komponenten, steigt die Klemmkraft ungehindert mit zunehmender Belastung des Pfanneneinsatzes, so dass ausreichende Verklemmung der Komponenten gewährleistet ist.
2. Tritt im rückseitigen Bereich Kontakt zwischen den Komponenten auf, so muss sichergestellt werden, dass der Kontakt nicht punktförmig auftritt, sondern einen Flächenkontakt darstellt. Punktkontakt führt zu lokalen Spannungsüberhöhungen, die zu Überlastung und frühzeitigen Ausfall führen. Ein Flächenkontakt ist allerdings bei den gezeigten Geometrien nicht sicherzustellen.
   Geringe Geometrieabweichungen, wie sie im Rahmen zulässiger Toleranzen immer möglich sind, sowie geringe Verkippungen in der Hüftpfanne durch asymmetrische Belastungen etc. führen bereits zu Punktkontakten und damit zu frühzeitigem Ausfall. Eigene Untersuchungen zeigen, dass ein kontrollierter Flächenkontakt im rückseitigen Bereich mit den vorhandenen Geometrien nicht möglich ist.

Es ist davon auszugehen, dass durch einen kontrollierten rückseitigen Kontakt der Komponenten unter Vermeidung bzw. Lösung der beschriebenen Probleme eine Reduktion der Zugspannungen im Pfanneneinsatz auftritt, was sich insbesondere bei der Verwendung keramischer Pfanneneinsätze positiv auswirkt. Die Bruchlast der Pfanneneinsätze würde dadurch signifikant erhöht. Bei Verwendung von Pfanneneinsätzen aus Metalllegierung ist zu erwarten, dass durch die Reduktion der Spannungen eine geringere Verformung auftritt. Dies kann dazu führen, dass die belastungsinduzierte Formabweichung der Gleitflächen reduziert wird und der Verschleiß der Gleitpartner abnimmt.

Eigene Untersuchungen unterstreichen die Notwendigkeit des rückseitigen Spaltes zwischen Komponenten mit dieser oder ähnlicher Geometrie.

Der Erfindung liegt die Aufgabe zugrunde, eine Hüftpfanne und Pfanneneinsatz für eine Hüftgelenkprothese nach dem Oberbegriff des Anspruchs 1 so zu verbessern, dass eine Reduktion der Zugspannungen im Pfanneneinsatz erreicht ist.

Erfindungsgemäß wird diese Aufgabe durch die Merkmale des Anspruchs 1 gelöst.

Dadurch, dass die radialen Konturzüge der beiden Komponenten, ausgehend vom unteren Konusende bis zum Pol, gleiche Geometrie-Elemente in gleicher Abfolge aufweisen und zwischen den Geometrie-Elementen tangentiale bzw. nahezu tangentiale Übergänge auftreten, ist eine Reduktion der Zugspannungen im Pfanneneinsatz erreicht.

In bevorzugter Ausführungsform ist der Spalt ein initialer Spalt ist, der im unbelasteten Zustand des Pfanneneinsatzes am größten ist und bei Lastaufbringung auf den Pfanneneinsatz abnimmt und ab einer bestimmten Last zumindest partiell geschlossen ist, so dass ein Kontakt der Komponenten auch unterhalb des Klemmkonus stattfindet. Die Bruchlast des Pfanneneinsatzes ist durch einen kontrollierten rückseitigen Kontakt dadurch signifikant erhöht.

Bevorzugt ist die Breite des initialen Spaltes zwischen den Komponenten im unbelasteten Zustand des Pfanneneinsatzes im konusnahen Bereich kleiner oder gleich der Breite des Spalts im Bereich des Pols.

Alternativ nimmt die Breite des initialen Spaltes zwischen den Komponenten im unbelasteten Zustand des Pfanneneinsatzes, ausgehend vom konusnahen Bereich hin zum Pol kontinuierlich zu.

Bevorzugt besteht die Hüftpfanne aus Metall und ist dünnwandig und damit besonders nachgiebig ausgebildet. Hierdurch kann sich bei Lastaufbringung auf den Pfanneneinsatz die Hüftpfanne aufweiten und der Pfanneneinsatz auf dem Klemmkonus in das Innere der Hüftpfanne gleiten bis ab einer bestimmten Last ein Kontakt der Komponenten auch unterhalb des Klemmkonus stattfindet.

Vorteilhaft besteht der Pfanneneinsatz aus Keramik und vorzugsweise aus einer Aluminiumoxidkeramik oder Mischkeramiken auf der Basis von Aluminiumoxid oder Zirkonoxid oder aus einer Silizium-Nitrid-Keramik.

In einer Ausführungsform der Erfindung ist die Rückseite des Pfanneneinsatzes genauso wie die innere Geometrie der Hüftpfanne als Teil einer Kugelsphäre ausgebildet.

In einer Ausführungsform ist außer am Klemmkonus der Radius R_{Einsatz-Rückseite} (R_{ER}) der Rückseite des Pfanneneinsatzes gleich oder größer als der Radius R_{Pfanne-Pol} (R_{PP}) der inneren Geometrie der Hüftpfanne.

Bevorzugt ist am Übergang zwischen Klemmkonus und Kugelsphäre der Verrundungsradius (R_{Einsatz-Verrundung}) der Rückseite des Pfanneneinsatzes gleich dem Verrundungsradius (R_{Pfanne-Verrundung}) der inneren Geometrie der Hüftpfanne.

Eine bevorzugte Ausführungsform ist dadurch gekennzeichnet, dass am Übergang zwischen Klemmkonus und Kugelsphäre jeweils ein Verrundungsradius besteht und R_{Einsatz-Rückseite} (R_{ER}) annähernd R_{Pfanne-Pol} (R_{PP}) und R_{Einsatz-Verrundung} (R_{EV}) größer R_{Pfanne-Verrundung} (R_{PV}) ist, der Pfanneneinsatz aus Aluminiumoxid-Mischkeramik besteht und die Hüftpfanne eine dünnwandige Metallpfanne ist. Zur Vermeidung der weiter oben beschriebenen Probleme und zur Erzielung eines sogenannten kontrollierten rückseitigen Kontaktes bzw. auch "kontrollierter Bodenkontakt" wird folgende Lösung vorgeschlagen:
Die rückseitige Geometrie des Pfanneneinsatzes und die innere Geometrie der Hüftpfanne werden so aufeinander abgestimmt, dass
   - auf einem radialen Konturzug, ausgehend vom unteren Konusende bis zum Pol der jeweiligen Komponente, nur tangentiale bzw. nahezu tangentiale Übergänge zwischen Geometrie-Elementen auftreten
   - die radialen Konturzüge, ausgehend vom unteren Konusende bis zum Pol, der zusammengehörigen Komponenten nur gleiche Geometrie-Elemente aufweisen, wobei die Dimensionen nicht gleich sein müssen
   - der initiale Spalt zwischen den Komponenten, d.h. im unbelasteten Zustand, im konusnahen Bereich kleiner oder gleich dem Spalt im Bereich des Pols ist
Nachfolgend wird die Erfindung anhand von drei Figuren näher erläutert.

Figur 1 zeigt eine Hüftgelenkprothese 12 nach dem Stand der Technik, die modular aufgebaut ist. Die Hüftgelenkprothese 12 besteht aus einem Schaft 1 gekoppelt mit einem Kugelkopf 2 und einer Hüftpfanne 4 gekoppelt mit einem Pfanneneinsatz 3. Schaft 1 und Hüftpfanne 4 sind in der Regel aus Metalllegierungen und mit dem Körper durch Einwachsen in den Oberschenkel- bzw. Beckenknochen verbunden. Sie sind Träger für den Kugelkopf 2 bzw. Pfanneneinsatz 3. Der Kugelkopf 2 ist in der Kalotte des Pfanneneinsatzes 3 drehbar gelagert mit dem Freiheitsgrad eins.

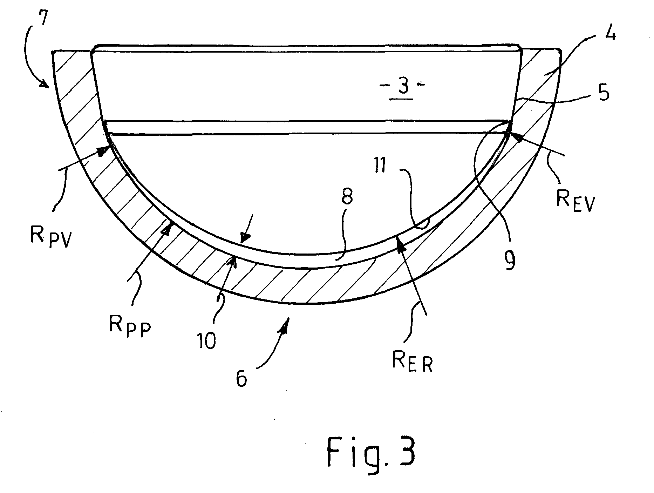

Eine bevorzugte Ausführungsform eines erfindungsgemäßen Pfanneneinsatzes und einer Hüftpfanne für eine Hüftgelenkprothese 12 (siehe Figur 1) ist in Figur 2 und 3 dargestellt. Der Pfanneneinsatz 3 ist mit einem Klemmkonus 5 in der Hüftpfanne 4 verankert. Mit dem Bezugszeichen 7 ist der äquatoriale Bereich und mit dem Bezugszeichen 6 der Pol gekennzeichnet. In dieser Ausführungsform sind die rückseitige Geometrie des Pfanneneinsatzes 3 und die innere Geometrie der Hüftpfanne 4 als Kugelsphären bzw. Teil von Kugelsphären ausgeführt. Am Übergang zum Klemmkonus 5 besteht jeweils eine Verrundung R_{EV} und R_{PV}. Der Radius R_{Einsatz-Rückseite} R_{ER} der Kugelsphäre der Rückseite 11 des Pfanneneinsatzes 3 ist geringfügig größer gestaltet als der Radius R_{Pfanne-Pol} R_{PP} der inneren Geometrie der Hüftpfanne 4. Der Verrundungsradius R_{Einsatz-Verrundung} R_{EV} am Pfanneneinsatz 4 ist gleich dem Verrundungsradius R_{Pfanne-Verrundung} R_{PV} der inneren Geometrie der Hüftpfanne 4. Der sich so ergebende Spalt 8 zwischen den Komponenten 3, 4 nimmt ausgehend vom Bereich nahe des unteren Konusendes 9 zum Pol 6 der Komponenten 3, 4 hin zu. Die Breite des Spalts 8 ist mit dem Bezugszeichen 10 gekennzeichnet.

## Patentansprüche

1. Hüftpfanne (4) und Pfanneneinsatz (3) für eine Hüftgelenkprothese (12), wobei der Pfanneneinsatz (3) mit der Hüftpfanne über einen Klemmkonus (5) einer konischen Klemmverbindung im äquatorialen Bereich (7) der beiden Komponenten (3, 4) gekoppelt ist und im unbelasteten Zustand des Pfanneneinsatzes (3) unterhalb des Klemmkonus (5) bis hin zum Pol (6) ein Spalt (8) zwischen den beiden Komponenten (3, 4) angeordnet ist, der von den radialen Konturzügen beider Komponenten (3, 4) begrenzt ist, **dadurch gekennzeichnet, dass** die radialen Konturzüge der beiden Komponenten (3, 4), ausgehend vom unteren Konusende (9) bis zum Pol (6), gleiche Geometrie-Elemente in gleicher Abfolge aufweisen und zwischen den Geometrie-Elementen tangentiale Übergänge auftreten.

2. Hüftpfanne und Pfanneneinsatz nach Anspruch 1, **dadurch gekennzeichnet, dass** der Spalt (8) ein initialer Spalt (8) ist, der im unbelasteten Zustand des Pfanneneinsatzes (3) am größten ist und bei Lastaufbringung auf den Pfanneneinsatz (3) abnimmt und ab einer bestimmten Last zumindest partiell geschlossen ist, so dass ein Kontakt der Komponenten (3, 4) auch unterhalb des Klemmkonus (5) stattfindet.

3. Hüftpfanne und Pfanneneinsatz nach Anspruch 2, **dadurch gekennzeichnet, dass** die Breite (10) des initialen Spaltes (8) zwischen den Komponenten (3, 4) im unbelasteten Zustand des Pfanneneinsatzes (3) im konusnahen Bereich kleiner oder gleich der Breite des Spalts im Bereich des Pols (6) ist.

4. Hüftpfanne und Pfanneneinsatz nach Anspruch 2, **dadurch gekennzeichnet, dass** die Breite (10) des initialen Spaltes (8) zwischen den Komponenten (3, 4) im unbelasteten Zustand des Pfanneneinsatzes (3), ausgehend vom konusnahen Bereich hin zum Pol (6) kontinuierlich zunimmt.

5. Hüftpfanne und Pfanneneinsatz nach einem der Ansprüche 1 bis 4, **dadurch gekennzeichnet, dass** die Hüftpfanne (4) aus Metall besteht und dünnwandig und damit besonders nachgiebig ausgebildet ist.

6. Hüftpfanne und Pfanneneinsatz nach einem der Ansprüche 1 bis 5, **dadurch gekennzeichnet, dass** der Pfanneneinsatz (3) aus Keramik besteht und vorzugsweise aus einer Aluminiumoxidkeramik oder Mischkeramiken auf der Basis von Aluminiumoxid oder Zirkonoxid oder aus einer Silizium-Nitrid-Keramik.

7. Hüftpfanne und Pfanneneinsatz nach einem der Ansprüche 1 bis 6, **dadurch gekennzeichnet, dass** die Rückseite (11) des Pfanneneinsatzes (3) genauso wie die innere Geometrie der Hüftpfanne (4) als Teil einer Kugelsphäre ausgebildet ist.

8. Hüftpfanne und Pfanneneinsatz nach Anspruch 7, **dadurch gekennzeichnet, dass** außer am Klemmkonus (5) der Radius R_{Einsatz-Rückseite} (R_{ER}) der Rückseite (11) des Pfanneneinsatzes (3) gleich oder größer als der Radius R_{Pfan-ne-Pol} (R_{PP}) der inneren Geometrie der Hüftpfanne (4) ist.

9. Hüftpfanne und Pfanneneinsatz nach Anspruch 7 oder 8, **dadurch gekennzeichnet, dass** am Übergang zwischen Klemmkonus (5) und Kugelsphäre der Verrundungsradius R_{Einsatz-Verrundung} (R_{EV}) der Rückseite (11) des Pfanneneinsatzes (3) gleich dem Verrundungsradius R_{Pfanne-Verrundung} (R_{PV}) der inneren Geometrie der Hüftpfanne (4) ist.

10. Hüftpfanne und Pfanneneinsatz nach einem der Ansprüche 7 bis 9, **dadurch gekennzeichnet, dass** am Übergang zwischen Klemmkonus (5) und Kugelsphäre jeweils ein Verrundungsradius besteht und R_{Einsatz-Rückseite} (R_{ER}) annähernd R_{Pfanne-Pol} (R_{PP}) und R_{Einsatz-Verrundung} (R_{EV}) größer R_{Pfanne-Verrundung} (R_{PV}) ist, der Pfanneneinsatz aus Aluminiumoxid-Mischkeramik besteht und die Hüftpfanne (4) eine dünnwandige Metallpfanne ist.

## Claims

1. A hip socket (4) and a socket insert (3) for a hip-joint prosthesis (12), wherein the socket insert (3) is coupled to the hip socket by way of a clamping cone (5) of a conical clamping connection in the equatorial region (7) of the two components (3, 4), and, in the unloaded state of the socket insert (3), beneath the clamping cone (5) as far as the pole (6) a gap (8) is arranged between the two components (3, 4) that is delimited by the radial contour lines of the two components (3, 4), **characterised in that** the radial contour lines of the two components (3, 4), starting from the lower cone end (9) as far as the pole (6), have the same geometric elements in the same sequence, and tangential transitions occur between the geometric elements.

2. A hip socket and socket insert according to claim 1, **characterised in that** the gap (8) is an initial gap (8) that is largest in the unloaded state of the socket insert (3) and decreases when a load is applied to the socket insert (3) and is at least partially closed from a certain load on so that a contact of the components (3, 4) also occurs beneath the clamping cone (5).

3. A hip socket and socket insert according to claim 2, **characterised in that** the width (10) of the initial gap (8) between the components (3, 4) in the unloaded state of the socket insert (3) in the region close to the cone is smaller than or equal to the width of the gap in the region of the pole (6).

4. A hip socket and socket insert according to claim 2, **characterised in that** the width (10) of the initial gap (8) between the components (3, 4) in the unloaded state of the socket insert (3) increases continuously starting from the region close to the cone towards the pole (6).

5. A hip socket and socket insert according to one of claims 1 to 4, **characterised in that** the hip socket (4) consists of metal and is formed with thin walls and thus so as to be particularly flexible.

6. A hip socket and socket insert according to one of claims 1 to 5, **characterised in that** the socket insert (3) consists of ceramic material and preferably of an aluminium-oxide ceramic material or mixed ceramic materials based on aluminium oxide or zirconium oxide or of a silicon-nitride ceramic material.

7. A hip socket and socket insert according to one of claims 1 to 6, **characterised in that** the rear side (11) of the socket insert (3) is formed in exactly the same way as the inner geometry of the hip socket (4) as part of a ball sphere.

8. A hip socket and socket insert according to claim 7, **characterised in that** apart from at the clamping cone (5) the radius Rᵢₙₛₑᵣₜ rear side (R_{ER}) of the rear side (11) of the socket insert (3) is equal to or greater than the radius R_{socket} pole (R_{PP}) of the inner geometry of the hip socket (4).

9. A hip socket and socket insert according to claim 7 or 8, **characterised in that** at the transition between the clamping cone (5) and the ball sphere the rounding radius Rᵢₙₛₑᵣₜ rounding (R_{EV}) of the rear side (11) of the socket insert (3) is equal to the rounding radius R_{socket} rounding (R_{PV}) of the inner geometry of the hip socket (4).

10. A hip socket and socket insert according to one of claims 7 to 9, **characterised in that** at the transition between the clamping cone (5) and the ball sphere in each case there is a rounding radius, and Rinsert rear side (R_{ER}) is approximately R_{socket} pole (R_{PP}) and Rinsert rounding (R_{EV}) greater than R_{socket} rounding (R_{PV}), the socket insert consists of aluminium-oxide mixed ceramic material, and the hip socket (4) is a thin-walled metal socket.

## Revendications

1. Cavité cotyloïde (4) et insert de cavité cotyloïde (3) pour une prothèse d'articulation de la hanche (12), où l'insert de cavité cotyloïde (3) est couplé à la cavité cotyloïde par l'intermédiaire d'un cône de serrage (5) d'un assemblage par serrage conique dans la région équatoriale (7) des deux composants (3, 4) et, à l'état sans charge de l'insert de cavité cotyloïde (3), il est prévu entre les deux composants (3, 4), au-dessous du cône de serrage (5), jusqu'au pôle (6), un interstice (8) qui est délimité par les tracés de contour radiaux des deux composants (3, 4), **caractérisés en ce que**, partant de l'extrémité de cône inférieure (9) jusqu'au pôle (6), les tracés de contour radiaux des deux composants (3, 4) présentent des éléments de géométrie identiques dans une succession identique, et qu'il existe des transitions tangentielles entre les éléments de géométrie.

2. Cavité cotyloïde et insert de cavité cotyloïde selon la revendication 1, **caractérisés en ce que** l'interstice (8) est un interstice initial (8) qui est le plus grand à l'état sans charge de l'insert de cavité cotyloïde (3) et diminue en cas d'application d'une charge à l'insert de cavité cotyloïde (3) et est au moins partiellement fermé à partir d'une charge définie, de sorte qu'il y a contact des composants (3, 4), même au-dessous du cône de serrage (5).

3. Cavité cotyloïde et insert de cavité cotyloïde selon la revendication 2, **caractérisés en ce que**, à l'état sans charge de l'insert de cavité cotyloïde (3), la largeur (10) de l'interstice initial (8) entre les composants (3, 4), dans la zone proche du cône, est inférieure ou égale à la largeur de l'interstice dans la région du pôle (6).

4. Cavité cotyloïde et insert de cavité cotyloïde selon la revendication 2, **caractérisés en ce que**, à l'état sans charge de l'insert de cavité cotyloïde (3), la largeur (10) de l'interstice initial (8) entre les composants (3, 4) augmente de façon continue à partir de la zone proche du cône, en direction du pôle (6).

5. Cavité cotyloïde et insert de cavité cotyloïde selon l'une des revendications 1 à 4, **caractérisés en ce que** la cavité cotyloïde (4) est en métal et est réalisée avec une paroi mince et donc de façon particulièrement souple.

6. Cavité cotyloïde et insert de cavité cotyloïde selon l'une des revendications 1 à 5, **caractérisés en ce que** l'insert de cavité cotyloïde (3) est en céramique et de préférence en céramique d'oxyde d'aluminium ou en céramiques mixtes à base d'oxyde d'aluminium ou d'oxyde de zirconium ou en céramique au nitrure de silicium.

7. Cavité cotyloïde et insert de cavité cotyloïde selon l'une des revendications 1 à 6, **caractérisés en ce que** la face arrière (11) de l'insert de cavité cotyloïde (3), tout comme la géométrie interne de la cavité cotyloïde (4), est réalisée en tant que partie d'une sphère.

8. Cavité cotyloïde et insert de cavité cotyloïde selon la revendication 7, **caractérisés en ce que**, à l'exception du cône de serrage (5), le rayon R_{insert-face} arrière (R_{ER}) de la face arrière (11) de l'insert de cavité cotyloïde (3) est égal ou supérieur au rayon R_{cavité} cotyloïde-pôle (R_{PP}) de la géométrie interne de la cavité cotyloïde (4).

9. Cavité cotyloïde et insert de cavité cotyloïde selon la revendication 7 ou 8, **caractérisés en ce que**, à la transition entre le cône de serrage (5) et la sphère, le rayon de raccordement R_{insert-raccordement} (R_{EV}) de la face arrière (11) de l'insert de cavité cotyloïde (3) est égal au rayon de raccordement R_{cavité} cotyloïde-raccordement (R_{PV}) de la géométrie interne de la cavité cotyloïde (4).

10. Cavité cotyloïde et insert de cavité cotyloïde selon l'une des revendications 7 à 9, **caractérisés en ce que**, à la transition entre le cône de serrage (5) et la sphère, il existe respectivement un rayon de raccordement, et R_{insert-face} arrière (R_{ER}) est approximativement Rcavité cotyloïde-pôle (R_{PP}) et R_{insert-raccordement} (R_{EV}) est supérieur à Rcavité cotyloïde-raccordement (R_{PV}), que l'insert de cavité cotyloïde est en céramique mixte d'oxyde d'aluminium et la cavité cotyloïde (4) est une cavité cotyloïde métallique à paroi mince.
